# EUROPEAN PATENT SPECIFICATION

(11) **EP 0 901 776 B1**
(45) Date of publication and mention of the grant of the patent: **24.09.2003**
(21) Application number: 98830260.0
(22) Date of filing: 29.04.1998
(51) Int. Cl.: A61F 2/06, A61M 25/10

(54) **System for introducing and positioning expandable stents**
System zur Einführung und Positionierung aufweitbarer Stents
Système pour introduire et positionner des stents expansibles

(30) Priority: 05.09.1997 IT BS970077
(43) Date of publication of application: 17.03.1999
(73) Proprietor: Invatec S.r.l, 25062 Concesio (Brescia) (IT)
(72) Inventor: Venturelli, Andrea, 25062 Concesio (Brescia) (JP)
(74) Representative: Manzoni, Alessandro

(56) References cited:
- EP-A- 0 707 837
- EP-A- 0 770 366
- WO-A-96/12517
- US-A- 4 776 337
- US-A- 5 514 093

## Description

The present invention pertains to the instruments for the introduction and the positioning of mechanically dilating stent in the ducts or lumina of a live, either human or animal body.

Stents are tubular molds which are made of biocompatible materials and are contracted on their introduction and then they dilate for their stable insertion into the desired duct or lumen. Some types of stents dilate mechanically, and for their insertion they require the use of an expandable element arranged along the introducing instrument and acting inside the stent.

As the instrument for the introduction of mechanically dilating steals, a catheter having an inflatable balloon at its distal end of the type already used may also be used, e.g., for dilating arterial ducts or other lumina in a live body. Therefore, for the introduction operation, the balloon, empty, is wrapped tightly around a corresponding zone of the catheter, and the stent, in its turn, is arranged tightly around the balloon in order to remain fixed there during the introduction into a lumen.

In general then, the catheter, at the balloon, has a radiopaque marker in order to be able to control the position of the catheter and, therefore, that of the balloon in the duct or lumen in question.

However the requirements of a catheter for the simple dilation of ducts or lumina *most* often are incompatible with those of a catheter for the positioning of a stent, which is why specific catheters would be needed for one or the other operation.

A dilating catheter, for example, for angioplasty procedures, or the like, must have a small diameter, a low coefficient of surface friction, and a highly resistant balloon. On the other hand, a catheter for the positioning of stents must have a remarkable friction, at least at the balloon, be made of a material having a surface that is adapted to the interior of the closed, i.e., contracted stent, and have a diameter of the balloon, when closed, i.e., wrapped around the catheter, that is at least slightly greater than the internal diameter of the closed stent. All this is to hold the stent and to prevent its loss during the insertion in a duct or lumen.

Otherwise, a diameter in the zone of the catheter, including the closed balloon wrapped there, that is smaller than the internal diameter of the closed stent that is applied there may cause:
- a so-called overlapping resulting from a corrugation and an overlapping of some parts of the stent with possible deformation of its structure, if the stent is contracted too tightly in order to adhere to such a zone; or
- an improper fixing of the stent with the possibility of losing it during the introduction if the stent, although suitably and correctly closed, does not adhere to the too-small outer surface of the catheter plus closed balloon.

Starting from these representative premises of the state of the art, the object of the present invention is to provide a valid solution to the problems mentioned above and to correspondingly provide an improved catheter structure, which is reliable for a correct arrangement of the stent contracted around the closed balloon and which makes it possible to reduce, if not to eliminate, the possibilities of release and loss of the stent during the phase of introduction into the duct or lumen in which it is inserted.

Another object of the present invention is to propose a catheter structure that can be used advantageously either as a catheter for dilating or a catheter for positioning expandable stents.

These objects are accomplished with a catheter structure for positioning stents according to claim 1. The features of the preamble are known from the prior art such as EP-A-0 770 366 or EP-A-707 837.

In practice, the contracted stent is thus arranged and is held positively between two shoulders, prevented from sliding axially and thus from being lost, and this is independent of the actual diameter of the catheter, of the material with which the balloon is made and of the degree of friction offered by the said balloon.

In addition, the diameter of the catheter portion between shoulders is adapted by increasing it with the application of the tubular adapter.

Greater details of the present invention, as well as its other aspects and advantages, shall become more evident from the description below with reference to the attached drawings, in which:
Figure 1 shows the partial view of a catheter according to the present invention with wrapped balloon and stent contracted around the balloon between two conical collars;
Figure 2 shows a similar view of the catheter of Figure 1, but with the balloon inflated and the stent dilated;
Figure 3 shows part of a catheter with biconical collars; and
Figures 4 and 5 show views that are similar to those of Figures 1 and 2, but in which the collars are defined by spherical elements.

in the said drawings, the catheter is indicated globally by 11 and has, in the known manner, an inflatable balloon 13 on its distal section 12.

Two collars 14, which are spaced apart and delimit two annular shoulders 15, one in front of the other, around the catheter, arc applied on the catheter part that is within the balloon.

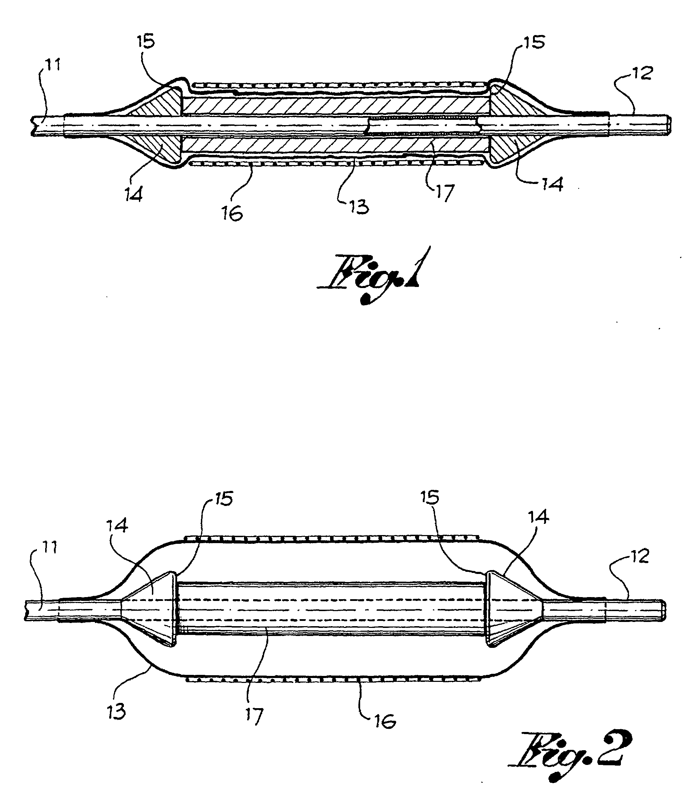

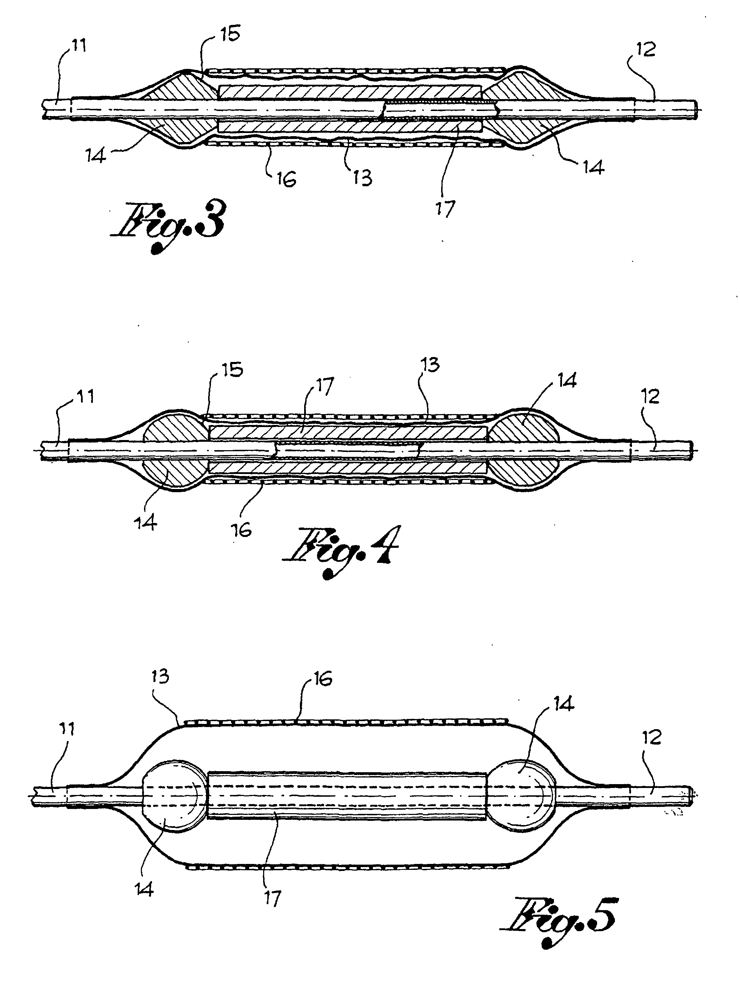

The two collars 14 may preferably be conical, with opposite conicities, as shown in Figures 1 and 2, one towards the distal end 12 of the catheter and the other in the opposite direction, in order to facilitate the introduction and the extraction of the catheter in a duct or lumen of a body. As an alternative, the said collars 14 may be biconical as shown in Figure 3, or spherical as shown in Figures 4 and 5.

The said collars 14 may be made of a radiopaque material in order to form markers which make it possible to find the position of the catheter in the duct or lumen, in which it is inserted.

The distance between the two collars and, therefore, between the annular shoulders 15 is at least slightly greater than the length of a stent 16 to be used and to be inserted with the dilating catheter described.

Therefore, to use this catheter in the positioning of an expandable stent 16, the empty balloon is wrapped tightly on the collars 14 and around the part of the catheter comprised between said collars. The stent 16 is then placed and contracted around the balloon and is held in the space comprised between the two annular shoulders 15 defined by the collars 14 as shown in Figures 1, 2 and 4.

If the catheter plus the balloon wrapped thereon has a suitable diameter, the contracted stent is adapted to the outer surface of the closed balloon with no overlapping and, moreover, positively held axially between the shoulders 15 and radially within the peripheral outline of the collars.

In the case of a particularly thin catheter, as in the catheters used for angioplasty, and in which the external diameter of the catheter plus the empty balloon wrapped thereon may be much smaller than the internal diameter of the contracted, i.e., closed, stent, without overlapping around the catheter part comprised between the two collars 14, the tubular adapter 17 held between the two shoulders 15 is applied and so as to artificially increase the diameter of the catheter as shown in the drawings. Therefore, the stent may be applied correctly on the catheter without the possibility of loss during the introduction into the duct or lumen in question.

Figures 2 and 5 of the drawings illustrate the condition of expansion of the balloon and of dilation of the stent for the release of same at the time of its insertion.

## Claims

1. Catheter structure for dilating and for positioning stents, comprising a catheter having a inflatable balloon,
two collars (14) fixed around the catheter part inside the said balloon and defining two annular shoulders (15) one in front of the other and spaced apart in parallel; the shoulders forming bilateral axial closing means for a stent that is arranged and contracted around the empty and wrapped balloon,
**characterized in that**
a tubular adapter (17) is arranged around the catheter portion between the said collars and increases the external diameter of said portion,
and **in that** the said balloon, when empty, is wrapped around the collars with the tubular adapter comprised between same.

2. Catheter structure in accordance with claim 1 in which the collars are conical with opposite conicities, one towards a distal end of the catheter and the other in the opposite direction.

3. Catheter structure in accordance with claim 1 in which the said collars are each biconical.

4. Catheter structure in accordance with claim 1 in which the said collars are spherical.

5. Catheter structure in accordance with the claims 2-4 in which the said collars are made of a radiopaque material.

6. Catheter structure in accordance with claim 1 in which the tubular adapter (17) is held between the said shoulders.

## Patentansprüche

1. Katheterstruktur zur Dilatation und Positionierung von Stents, einschließlich eines Katheters mit aufblasbarem Ballon,
zwei Kragen (14), die um den Katheter herum auf der Innenseite des besagten Ballons befestigt sind und zwei ringförmige Schultern (15) bilden, eine an der Front und eine parallel versetzt; die Schultern bilden einen zweiseitigen axialen Abschluss für einen Stent, der positioniert und um den leeren und aufliegenden Ballon zusammengezogen wird;
**dadurch gekennzeichnet, dass** ein rohrförmiger Adapter (17) um den Katheterteil herum zwischen den besagten Kragen angebracht ist und den Außendurchmesser des besagten Teils erweitert,
**dadurch gekennzeichnet, dass** der besagtem Ballon im leeren Zustand um die Kragen mit dem dazwischen liegenden rohrförmigen Adapter liegt.

2. Katheterstruktur gemäß Anspruch 1, wobei die Kragen konisch einander entgegengesetzter Konizität sind, einer zum distalen Ende des Katheters und der andere in Gegenrichtung.

3. Katheterstruktur gemäß Anspruch 1, wobei die Kragen jeweils bikonisch sind.

4. Katheterstruktur gemäß Anspruch 1, wobei die Kragen sphärisch sind.

5. Katheterstruktur gemäß der Ansprüche 2 - 4, wobei die Kragen aus radiopakem Material bestehen.

6. Katheterstruktur gemäß Anspruch 1, wobei der rohrförmige Adapter (17) zwischen den besagten Schultern gehalten wird.

## Revendications

1. Structure d'un cathéter pour la dilatation et le positionnement des stents, comprenant un cathéter doté d'un ballon gonflable, pourvu de deux colliers (14) fixés autour de la partie du cathéter située à l'intérieur du ballon et déterminant deux épaulements en forme d'anneau (15), placés l'un face à l'autre et séparés l'un de l'autre de façon parallèle, les épaulements constituant des procédés de fermeture axiale et bilatérale pour un stent, lequel est disposé et contracté autour du ballon vide ; **caractérisé par** un adaptateur tubulaire (17) disposé autour de la partie du cathéter située entre les colliers et augmentant le diamètre extérieur de ladite partie, et **caractérisé par le fait que** le ballon, lorsqu'il est vide, est enveloppé par les colliers au moyen de l'adaptateur tubulaire compris entre ceux-ci.

2. Structure d'un cathéter selon la revendication 1, dans laquelle les colliers sont de forme conique - leurs conicités respectives étant opposées l'une par rapport à l'autre -, l'un étant situé à une extrémité du cathéter et l'autre dans la direction opposée.

3. Structure d'un cathéter selon la revendication 1, dans laquelle les colliers sont tous deux biconiques.

4. Structure d'un cathéter selon la revendication 1, dans laquelle les colliers sont sphériques.

5. Structure d'un cathéter selon les revendications 2-4, dans laquelle les colliers sont fabriqués en matériau radio-opaque.

6. Structure d'un cathéter selon la revendication 1, dans laquelle l'adaptateur tubulaire (17) est maintenu fixé entre les épaulements.
